# EUROPEAN PATENT SPECIFICATION

(11) **EP 2 344 664 B1**
(45) Date of publication and mention of the grant of the patent: **22.05.2013**
(21) Application number: 09752928.3
(22) Date of filing: 16.10.2009
(51) Int. Cl.: C12Q 1/22

(54) **BIOLOGICAL ARTICLES AND METHODS FOR MONITORING STERILIZATION PROCESSES**
BIOLOGISCHE ARTIKEL UND VERFAHREN ZUR KONTROLLE VON STERILISATIONSVORGÄNGEN
ARTICLES ET PROCÉDÉS POUR CONTRÔLER DES PROCÉDÉS DE STÉRILISATION

(30) Priority: 17.10.2008 US 196415 P
(43) Date of publication of application: 20.07.2011
(73) Proprietor: 3M Innovative Properties Company, Saint Paul, MN 55133-3427 (US)
(72) Inventor: CHANDRAPATI, Sailaja, Saint Paul, Minnesota 55133-3427 (US); WEBB, Heather M., Saint Paul, Minnesota 55133-3427 (US); HALVERSON, Kurt J., Saint Paul, Minnesota 55133-3427 (US)
(74) Representative: Vossius & Partner
(86) International application number: PCT/US2009/060936
(87) International publication number: WO 2010/045517

(56) References cited:
- EP-A2- 1 099 764
- DE-A1- 4 037 053
- US-A- 3 661 717
- US-A- 5 795 730
- US-A1- 2004 023 319

## Description

### BACKGROUND

Monitoring the effectiveness of processes used to sterilize equipment such as medical devices, instruments and other non-disposable articles is routinely carried out in health care as well as various industrial setting. An effective sterilization process is expected to completely destroy all viable microorganisms, including structures such as viruses and spores. Hospitals, as a standard practice to assay the lethality of a sterilization process, include a sterility indicator with a batch of articles to be sterilized. Both biological and chemical sterility indicators have been used.

A standard type of biological sterility indicator includes a known quantity of test microorganisms, for example *Geobacillus stearothermophilus* (formerly *Bacillus stearothermophilus* or *Bacillus atrophaeus* (formerly *Bacillus subtilis*) spores, which are many times more resistant to a sterilization process than most contaminating organisms. After the indicator is exposed to the sterilization process, the spores are incubated in nutrient medium to determine whether any of the spores survived the sterilization process, with spore growth indicating that the sterilization process was insufficient to destroy all of the microorganisms. In another example, after being subjected to a sterilization process, the activity of an enzyme, which can be correlated with spore viability, is determined. Although advances have been made; the time period for determining with certainty whether or not the sterilization process was effective can be undesirably long.

Available chemical sterility indicators can be read immediately at the end of the sterilization process. However, the results indicate only that a particular condition was present, such as the presence of a particular chemical or a temperature for a certain period of time.

It is generally considered that the response of living organisms to all conditions actually present is a more direct and reliable test for how effective a sterilization process is in achieving sterilization. Accordingly, there is a continuing need for biological sterility indicators which can indicate the effectiveness of a sterilization process without an excessive delay after completion of the sterilization process.

US 2004/0023319 discloses the rapid enumeration of viable spores by flow cytometry. US 5795730 discloses a rapid read-out biological indicator.

### SUMMARY

The present invention provides a sterility indicator including a composition, and a method of determining the effectiveness of a sterilization process using the indicator. The composition includes sterilization process resistant spores and a sub-lethal amount of at least one cell-permeant nucleic acid-interacting fluorescent dye in combination with at least one nutrient. In the event that any of the spores survive a sterilization process, the dye can interact with nucleic acids present at least during germination (for example, during germination or during germination and outgrowth) of the spores, thereby causing an increase in fluorescence intensity. In certain embodiments, an increase in nucleic acid content, if present, and the resulting increase in fluorescence intensity can be detected during or after a short incubation time. In certain embodiments, such incubation may be carried out with the spores in contact with a germination medium containing the combination of fluorescence dye and at least one nutrient. The cell-permeant nucleic acid-interacting fluorescent dye is sufficiently stable at least at a temperature for incubating the spores to produce the increase in fluorescence intensity. In certain embodiments, additionally the cell-permeant nucleic acid-interacting fluorescent dye is stable at sterilization process conditions routinely encountered by biological sterilization indicators.

In an embodiment, there is provided a sterilization process indicator comprising:
a carrier supporting a plurality of sterilization process resistant spores;
a container impermeable to microorganisms and impermeable to a sterilant, the container containing a germination medium comprising a sub-lethal amount of at least one cell-permeant nucleic acid-interacting fluorescent dye and at least one nutrient for germination of the spores;
wherein the at least one cell-permeant fluorescent dye can interact with nucleic acids present in and produced by the plurality of spores during germination or during germination and outgrowth of the spores to produce an increase in fluorescence intensity compared with a fluorescence intensity prior to germination of the spores, indicating that viable spores are present, and wherein the cell-permeant fluorescent dye is sufficiently stable at least at a temperature for incubating the spores to produce the increase in fluorescence intensity.
wherein the carrier is adjacent the container and separate from the germination medium.

In a further embodiment, there is provided a method of determining the effectiveness of a sterilization process, the method comprising:
providing a sterilization process indicator comprising:
   a carrier supporting a plurality of sterilization process resistant spores;
   a container impermeable to microorganisms and impermeable to a sterilant, the container containing a germination medium comprising a sub-lethal amount of at least one cell-permeant nucleic acid-interacting fluorescent dye and at least one nutrient for germination of the spores;
   wherein the at least one cell-permeant fluorescent dye can interact with nucleic acids present in and produced by the plurality of spores during germination or during germination and outgrowth of the spores to produce an increase in fluorescence intensity compared with a fluorescence intensity prior to germination of the spores, indicating that viable spores are present, and wherein the cell-permeant fluorescent dye is sufficiently stable at least at a temperature for incubating the spores to produce the increase in fluorescence intensity; and
   wherein the carrier is adjacent the container and separate from the germination medium;
   positioning the sterilization process indicator in a sterilization chamber;
   exposing the sterilization process indicator to a sterliant;
   combining the plurality of sterilization process resistant spores and the germination medium;
   incubating the spores with the germination medium; and
   measuring the increase in fluorescence intensity, if present.

### Definitions

The term "sub-lethal amount" refers to an amount, for example, a concentration, of cell-premeant nucleic acid-interacting fluorescent dye that enables the production of sufficient fluorescent signal without adversely impacting the viability of the spores, germinating spores or vegetative cells. With the sufficient fluorescent signal, the increase in fluorescence intensity, if present, can be detected.

The term "cell-permeant" refers to a dye that is passively or actively transported or able to pass through the spore coat and/or cell membrane sufficiently to contact nucleic acids in the spores, germinating spores, or vegetative cells without the aid of any agent used to make the spore coat and/or cell membrane more permeable to the dye molecules.

The numbers, E5, E6, and E7 are used interchangeably herein with 10⁵, 10⁶, and 10⁷, respectively.

The term "comprising" and variations thereof (e.g., comprises, includes, etc.) do not have a limiting meaning where these terms appear in the description and claims.

As used herein, "a," "an," "the," "at least one," and "one or more" are used interchangeably, unless the context clearly dictates otherwise.

Also herein, the recitations of numerical ranges by endpoints include all numbers subsumed within that range (e.g., 500 to 7000 nm includes 500, 530, 551, 575, 583, 592, 600, 620, 650, 700, etc.).

The above summary of the present invention is not intended to describe each disclosed embodiment or every implementation of the present invention. The description that follows more particularly exemplifies illustrative embodiments.

### BRIEF DESCRIPTIONS OF THE FIGURE

FIG. 1 is a cross-sectional view of one embodiment of a sterility indicator of the present invention, with cap 26 not present.
FIG. 2 is an exploded perspective view of the sterility indicator of FIG. 1, cap 26 included.

### DETAILED DESCRIPTION OF ILLUSTRATIVE EMBODIMENTS OF THE INVENTION

As indicated above, biological sterility indicator compositions are now provided which can detect viable spores during germination or during germination and outgrowth of the spores, using a sub-lethal amount of at least one cell-permeant nucleic acid-interacting fluorescent dye and at least one nutrient for germination of the spores. The dye is stable at least at a temperature for incubating the spores. Such temperatures can be encountered when incubating the spores with the germination medium, which includes the at least one cell-permeant nucleic acid-interacting fluorescent dye and the at least one nutrient. For certain embodiments, preferably the dye is stable at sterilization temperatures, which may be significantly higher than a temperature for incubating the spores.

The biological sterility indicator compositions can be used advantageously in biological sterility indicators, such as those described herein. The compositions and indicators containing the compositions are useful in determining the effectiveness of a sterilization process, such as in the method, described herein, of determining the effectiveness of a sterilization process.

For certain embodiments, including any one of the indicator, or method embodiments described herein, the at least one cell-permeant fluorescent dye can interact with nucleic acids present in and produced by the plurality of spores during germination of the spores to produce the increase in fluorescence intensity. The interaction of the dye with the nucleic acids during germination provides an early indication of viable spores, if present.

For certain embodiments, including any one of the indicator, or method embodiments described herein, the at least one cell-permeant fluorescent dye can interact with nucleic acids present in and produced by the plurality of spores during germination and outgrowth of the spores to produce the increase in fluorescence intensity. Interaction of the dye with nucleic acids present also during outgrowth of the spores can also be useful, for example, by providing a greater increase in fluorescence when viable spores are present.

A number of sterilization process are presently known and in use, including, for example, exposure to steam, dry heat, gaseous or liquid agents such as ethylene oxide, hydrogen peroxide, peracetic acid, and ozone, and radiation. The plurality of sterilization process resistant spores may be selected according to the sterilization process to be used. Any spores may be used as long as they provide sufficient resistance to the sterilization process conditions, such that the spores are more resistant to the sterilization process conditions than most microorganisms encountered in natural contamination. For example, for a steam sterilization process, *Gb. stearothermophilus* (formerly *Bacillus stearothermopbilus)* may be used. In another example, for an ethylene oxide sterilization process, *B. atrophaeus* (formerly *B. subtilis*) may be used. For certain embodiments, including any one of the above composition, indicator, and method embodiments, the plurality of sterilization process resistant spores is selected from the group consisting of *Gb. stearothermophilus, B. atrophaeus, B. megaterium, Clostridium sporogenes, Bacillus coagulans,* and a combination thereof. For certain of these embodiments, the plurality of sterilization process resistant spores is selected from the group consisting of *Gb. stearothermophilus, B. atrophaeus, B. megaterium,* and a combination thereof.

By way of example only, the present disclosure describes the microorganisms used in the biological sterilization indicator as being "spores;" however, it should be understood that the type of microorganism (e.g., spore) used in a particular embodiment of the biological sterilization indicator is selected for being highly resistant to the particular sterilization process contemplated. Accordingly, different embodiments of the present disclosure may use different microorganisms, depending on the sterilization process for which the particular embodiment is intended.

Using cell-permeant nucleic acid-interacting fluorescent dyes, the present compositions, indicators, and methods can detect the presence of nucleic acids within the spores and an increase in nucleic acid content within the spores when such an increase occurs. During germination of the spores, the shift in metabolic state of the spores from dormancy to growth requires a concomitant increase in nucleic acid content.

Cell-permeant fluorescent dyes are known to be toxic or mutagenic to microorganisms. However, it has now been found that cell-permeant dyes can be used in an amount which is sub-lethal to microorganisms and yet provides an increase in fluorescence during germination or during germination and outgrowth of the microorganisms. For certain embodiments, including any one of the above composition, indicator, and method embodiments, the concentration of the cell-permeant fluorescent dye in the medium is not more than 0.10 mM, 0.05 mM, or 0.01 mM. For certain of these embodiments, the concentration of the dye is not less than 0.0001 mM, 0.0005 mM, or 0.001 mM.

In some, if not all, of the sterilization processes in use, an elevated temperature, for example, 50 °C, 100 °C, 121 °C, 132 °C, 134 °C, or the like, is included or may be encountered in the process. Accordingly, for certain embodiments, including any one of the above composition, indicator, and method embodiments, the cell-permeant nucleic-acid-interacting fluorescent dyes are stable at sterilization temperatures.

For certain embodiments, including any one of the above indicator, and method embodiments, cell-permeant nucleic acid-interacting fluorescent dye is stable at a temperature up to at least 121 °C. For certain of these embodiments, the cell-permeant nucleic acid-interacting fluorescent dye is stable at a temperature up to at least 132 °C. For certain of these embodiments, the cell-permeant nucleic acid-interacting fluorescent dye is stable at a temperature up to at least 134 °C.

For certain embodiments, including any one of the above indicator, or method embodiments, the medium is essentially free of any background fluorescence at emission and excitation wavelengths used to detect the increase in fluorescence intensity. This may provide an improved sensitivity during germination or during germination and outgrowth of viable spores, if present, since any background level of fluorescence occurring at the same wavelengths as the emission and excitation of the dye interacting with a nucleic acid, is minimized.

For certain embodiments, including any one of the above indicator, or method embodiments, the medium is essentially free of any nucleic acids other than nucleic acids present in and produced by the plurality of spores. This may provide an improved sensitivity during germination or during germination and outgrowth of viable spores, if present, since any baseline level of fluorescence resulting from the dye interacting with any nucleic acids not produced by the plurality of spores is minimized.

The cell-permeant dye has a lower level of fluorescence at the emission wavelength (or wavelength range) when not interacting with a nucleic acid and a higher level fluorescence at this wavelength when interacting with a nucleic acid. For example, in certain embodiments, when not interacting with a nucleic acid the cell-permeant dye has a fluorescence quantum yield of less than 0.1, preferably less than 0.05, more preferably not more than 0.01. In certain of these embodiments, when interacting with a nucleic acid the cell-permeant dye has a fluorescence quantum yield of at least 0.1, preferably at least 0.2, more preferably at least 0.4.

For certain embodiments, including any one of the above indicator, or method embodiments, the cell-permeant fluorescent dye is a dye which interacts with DNA, RNA, or DNA and RNA. The interaction of the dye with DNA and RNA may be the same or different. The dye interacting with DNA may have a different excitation and/or emission maximum than the same dye interacting with RNA.

For certain embodiments, including any one of the above indicator, or method embodiments, the cell-permeant fluorescent dye is a dye which interacts with the nucleic acids in a variety of ways know in the art, including intercalation, electrostatic attraction, charge interaction, hydrophilic-hydrophobic interaction, or a combination thereof. As indicated above, this interacting or binding of the dye with nucleic acids, which include total cellular nucleic acids, such as DNA, RNA (mRNA, rRNA, tRNA), and extrachromosomal nucleic acids, causes a relatively large increase in fluorescence from the dye. For certain of these embodiments, the cell-permeant fluorescent dye is selected from the group consisting of acridine orange, a substituted unsymmetrical cyanine dye, and a combination thereof. Acridine orange bound to a DNA has an excitation maximum at about 490 nm and an emission maximum at about 520 maximum, but when bound to an RNA about 530 nm and 620 nm, respectively. See MacInnes, J. W and McClintock, M., Differences in Fluorescence Spectra of Acridine Orange-DNA Complexes Related to DNA Base Composition, Biopolymers. Communications to the editor, Vol 9, Pages 1407-1411 (1970). For certain embodiments, fluorescence at the emission maximum of the dye bound to RNA can be used to indicate germination of viable spores. This may be useful when the amount of RNA increases more rapidly than the amount of DNA in the germinating spores, allowing an earlier determination as to whether or not viable spores are present.

Suitable examples of substituted unsymmetrical cyanine dyes include dyes available under the trade name, SYTO (Invitrogen Corp., Carlsbad, CA). SYTO dyes are permeable to many, if not all, cell membranes but may differ from each other, for example, in degree of cell permeability, amount of fluorescence intensity increase when bound to a nucleic acid, excitation and emission maxima, selectivity in binding to DNA and RNA, and binding affinity to DNA and RNA. See Tamok, Cytometry Part A, 73A, 477-479 (2008). For certain embodiments, preferably the substituted unsymmetrical cyanine dye is SYTO 24 or SYTO 64. SYTO 24 has an excitation maximum at 490 nm and an emission maximum at 515 nm. SYRO 64 has an excitation maximum at 599 nm and an emission maximum at 619 nm.

For certain embodiments, including any one of the above indicator, and method embodiments, preferably the increase in fluorescence intensity is at a wavelength of 500 nm to 700 nm, preferably 500 nm to 675 nm. This wavelength range may be advantageous in that such fluorescence may not be obscured or may be significantly less obscured by absorbance or fluorescence of other components of the germination medium, which often occur at shorter wavelengths, such as wavelengths less than 500 nm or less than 400 nm.

The at least one nutrient induces germination and may also provide for outgrowth of the spores, if viable, with the simultaneous production of nucleic acids. Moreover, it has been found that the presence of the at least one nutrient with the dye reduces the effective toxicity of the dye with respect to the spores. The nutrient includes one or more sugars, for example, glucose, fructose, cellibiose, or the like. The nutrient may also include a salt such as potassium chloride, calcium chloride, or the like. The nutrient may also include at least one amino acid, for example, at least one of methionine, phenylalanine, and tryptophan. The germination medium may also include one or more other materials with the nutrient. The quantities of such nutrients and materials as well as other nutrients known in the art for inducing germination and initial outgrowth of the sterilization process resistant spores may be used. Media components and concentrations are known and described, for example, WO 99/05310 (Tautvydas) and Zechman et al., J. Food, Sci., 56, 5, p.1408-1411(1991), referred to as Zechman and Pflug, 1991.

For certain embodiments, including any one of the indicator, or method embodiments described herein, the germination medium further comprises a collisional quenching component. Such components reduce the background fluorescence signal of free (unbound to nucleic acid) cell-permeant dye through collisional quenching. Examples of species known to collisionally quench fluorescence include organic compounds such as purines, pyrimidines, aliphatic amines ,and nitroxides, certain ions, for example, nitrate anions and dissolved metal ions. Other species known to collisionally quench fluorescence are described, for example, in Principles of Fluorescence Spectroscopy, Chapter 9, Joseph R. Lakowicz, Plenum Press, 1983.

For certain embodiments, including any one of the indicator, or method embodiments described herein, the germination medium further comprises at least one reference dye. The reference dye does not bind to nucleic acids but responds similarly to cell-permeant dyes to changes in temperature or changes in the medium induced by spore germination and outgrowth, for example an increase or decrease in pH, ionic strength, or concentration change in metabolic by-products that alter the fluorescent signal. By monitoring the reference dye, signal from the cell-permeant dye binding to nucleic acid can be distinguished from signal produced from a change in temperature or a change in the media. The reference dye preferably fluoresces at a different wavelength than the cell-permeant dye.

The germination medium and the sterilization process resistant spores are kept separate but in close proximity to each other for ease of combining the medium and the spores when desired, for example, after exposure to a sterilization process and then incubating to determine whether or not any viable spores arc present as would be indicated by an increase in fluorescence intensity, or after exposure to a sterilization process but without incubating to determine a baseline or background fluorescence intensity. For example, a background level of fluorescence at a particular wavelength may be measured. Accordingly, for certain embodiments, including any one of the above composition, indicator, and method embodiments, the plurality of sterilization process resistant spores and the germination medium are separate from each other and adjacent each other.

For certain embodiments, including any one of the above indicator, and method embodiments, the germination medium is an aqueous solution or suspension. The at least one cell-permeant nucleic acid-interacting fluorescent dye and the at least one nutrient for germination of the spores can be dissolved or suspended in the aqueous medium. The concentration of the at least one cell-permeant nucleic acid-interacting fluorescent dye in the medium is dependent on the minimum level needed to provide an increase in fluorescence intensity during germination or during germination and outgrowth of the spores, and on the maximum level that can be tolerated by the spores without a significant adverse effect on the germination of the spores. Examples of dye concentrations that may be used are described above as well as in the Examples below. When viable spores are present the increase in fluorescence intensity can occur in less than 1 hour, preferably less than 30 minutes, more preferably less than 15 minutes.

In one alternative, for certain embodiments, the germination medium is in a dry form. The at least one cell-permeant nucleic acid-interacting fluorescent dye and the at least one nutrient can be dried together or separately to form a film or a layer on a support film or on a carrier material, optionally in a desired shape, or compounded together or separately as dry solids to form a tablet(s), caplet(s), or capsule(s). Any of these forms can be kept adjacent the spores, and water or an aqueous buffer can be added at an appropriate time to incubate the spores with the resulting medium suspension or solution. When resuspended or dissolved, the resulting medium can be a liquid or a gel. Additional embodiments of a medium in a dry form, which can be used in the composition, indicator, and method embodiments described herein are described in copending U.S. Patent Application Serial No. 61/196,438 (Chandrapati et al.), filed October 17, 2008, entitled Biological Sterilization Indicator, System, and Methods of Using Same.

When incubating the spores with the germination medium, an incubation temperature above room temperature may be used. For certain embodiments, the incubation temperature is at least 37 °C. For certain embodiments, preferably the incubation temperature is at least 50 °C. For certain embodiments, the incubation temperature is 50 to 60 °C.

As indicated above, the sterilization process indicator provided herein comprises a carrier supporting a plurality of sterilization process resistant spores. For certain embodiments, the carrier is a sheet material such as paper, woven cloth, nonwoven cloth, plastic, a polymeric material, a microporous polymeric material, metal foil, glass, porcelain, ceramic, or the like, or a combination thereof. For certain embodiments, the sheet material is water-absorbent or can be wetted to aid in quickly bringing the germination medium in intimate contact with the spores at the appropriate time.

The sterilization process indicator also comprises a container, which contains the germination medium without allowing the sterilant or any microorganism to enter the container. As indicated above, the carrier is adjacent to the container for ease of contacting the spores, supported by the carrier, with the germination medium when incubation is to be initiated. The container can be readily opened to contact the spores with the medium by expelling a plug, crushing or puncturing the container, or the like. The container can be equipped with a plug, or at least a portion of the container can be a breakable material, such as glass (e.g., glass ampoule) or other material, which can be breached by physical pressure but sufficiently tough to remain intact during manufacturing, storage, shipping, and sterilization conditions.

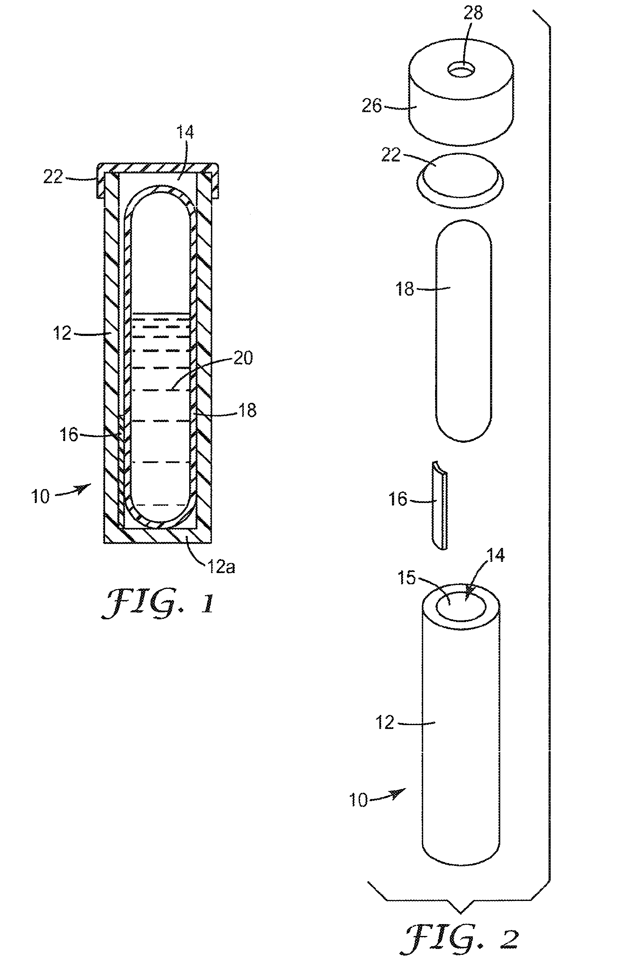

Known biological sterilization process indicator constructions such as those described in U.S. Patent No. 5,073,488 (Matner et al.) may be used with the above described compositions. Other indicator constructions may also be used, such as those described in U.S. Patent Application Serial No. 61/196,438 (Chandrapati et al.), filed October 17, 2008, entitled Biological Sterilization Indicator, System, and Methods of Using Same. One embodiment of the sterilization process indicator described herein is shown in FIGS. 1 and 2. The indicator includes a housing 10 having an open chamber 14 defined by gas and liquid impermeable walls 12. Alternative embodiments for these structures are shown in U.S. Patent Application Serial No. 61/196,438 (Chandrapati et al.), filed October 17, 2008, entitled Biological Sterilization indicator, System, and Methods of Using Same. The housing is shown as a circular tube, but other known configurations can be used. The walls are preferably transparent or translucent to the extent that a fluorescence intensity at a particular wavelength can be measured. Suitable materials for the walls may include glass, polycarbonate, polypropylene, polyester, and the like. For certain embodiments, at least one wall of the housing transmits at least 90 % of incident light within a wavelength range of at least 500 to 700 nm, preferably at least 500 to 675 nm. The chamber contains a carrier 16, for example, a paper, glass, or polymeric sheet, with a predetermined number of viable sterilization process resistant spores supported by the carrier.

Container 18, which holds germination medium 20, is shown within chamber 14. Alternatively, container 18 can be positioned outside of and adjacent chamber 14. Container 18, which is sealed, can be a breakable ampoule, but could alternatively be a container equipped with a plug, or other mechanism which when activated allows germination medium 20 to contact carrier 16 and the spores supported thereon. Container 18 is shown as an elongated ampoule, but other known configurations can be used as well.

Carrier 16 is shown between container 18 and wall 12 of housing 10 for ease of determining a change in fluorescence intensity through wall 12. Alternatively, a portion of wall 12 may be used as carrier 16. Other placements of carrier 16 may be used, for example, placement adjacent bottom wall 12A may be used. Carrier 16 may be shaped to fit this placement based upon the shape of housing 10, or bottom wall 12A may itself be used as carrier 16. For certain embodiments, carrier 16 transmits at least 90 % of incident light within a wavelength range of at least 500 to 700 nm, preferably at least 500 to 675 nm. Opening 15 to chamber 14 is provided with a gas-transmissive, microorganism-impermeable closure member 22, which may be adhered to housing 10 by an adhesive, a heat seal, or the like. Alternatively, closure member 22 may be held on to opening 15 with a cap 26 having an aperture 28. During exposure to a sterilant, the sterilant passes through the closure member 22, enters chamber 14, and contacts the spores on carrier 16. Alternative embodiments for these structures are shown in U.S. Patent Application Serial No. 61/196,438 (Chandrapati et al.), filed October 17, 2008, entitled Biological Sterilization Indicator, System, and Methods of Using Same.

The method provided herein of determining the effectiveness of a sterilization process includes providing any one of the above embodiments of a sterilization process indicator and positioning the sterilization process indicator in a sterilization chamber. Sterilizers, many of which are commercially available, include a sterilization chamber, which is typically sized to contain a plurality of articles to be sterilized, and equipped with a means of evacuating air and/or other gases from the chamber and adding a sterilant to the chamber. The biological sterilization indicator can be placed in the most difficult part in the sterilizer (e.g., above the drain). Alternately, the biological sterilization indicator can be placed adjacent an article to be sterilized when placed in the sterilization chamber. Additionally, the biological sterilization indicator can be adapted into routinely used process challenge devices before placing in a sterilizer.

The method includes exposing the sterilization process indicator to a sterilant. The sterilant can be added to the sterilization chamber after evacuating the chamber of at least a portion of any air or other gas present in the chamber. Alternatively, sterilant may be added to the sterilization chamber without evacuating the chamber. A series of evacuation steps is often used to assure that the sterilant reaches all areas within the sterilization chamber and contacts all areas of the article(s) to be sterilized. When the sterilant is added to the sterilization chamber, the sterilant also contacts the spores under conditions where the sterilant reaches all areas within the sterilization chamber.

The method also includes combining the plurality of sterilization resistant spores and the germination medium, thereby bringing the spores and germination medium into contact with each other. This may be done after the sterilization process is completed, that is, after conditions have been provided for the sterilant to reach all areas within the sterilization chamber for a time and at a temperature, believed to be sufficient to kill any microorganisms present within the sterilization chamber. The medium containing the cell-permeant fluorescent dye can be combined with the spores, and the combined spores and medium can be incubated, both as described above. The fluorescence intensity can be monitored and measured continuously or intermittently while incubating the spores with the germination medium.

For certain embodiments, including any one of the above method embodiments, the method further comprises determining whether or not viable spores are present, after exposing the sterilization process indicator to a sterilant, by measuring the increase in fluorescence intensity, if present, while incubating the spores with the germination medium and determining a rate of increase in fluorescence intensity, if present. For certain embodiments, sterilization resistant spore germination may be detected as a rate of fluorescence intensity increase over at least a portion of the incubation time. This rate may be linear, exponential, or the like, with the respect to incubation time. A rate constant can be calculated and used as an indicator of germination of the spores and, therefore, the presence of viable spores.

Viable spores, if present, on contact with the medium and incubation conditions, undergo a series of changes, including the *de Novo* production of DNA, RNA, and other extra chromosomal nucleic acids. The resulting detectable change in the dye interacting with the nucleic acids can be measured by measuring changes in optical properties such as fluorescence intensity at a particular wavelength. Such measurements can be conveniently carried out using known instruments such as a fluorometer, luminometer, or the like. For certain embodiments, preferably the detectable change is measured by measuring fluorescence intensity at a particular wavelength.

In one alternative, a portion or all of the incubating step may be carried out prior to measuring any increase in fluorescence intensity. For certain embodiments, including any one of the above method embodiments, other than where a rate of increase in fluorescence intensity is determined, the method further comprises determining whether or not viable spores are present, after exposing the sterilization process indicator to a sterilant, by measuring the increase in fluorescence intensity, if present, after incubating the spores with the germination medium as compared with before incubating the spores with the germination medium.

In another alternative, incubating may be carried with the cell-permeable nucleic acid-interacting dye separate from the at least on nutrient, wherein the spores are incubated with the medium containing the at least one nutrient, and subsequently adding medium containing the dye to the incubated spores. Any increase in fluorescence intensity above a threshold value may then be measured.

In the above method embodiments, optionally a fluorescence intensity of the combination of spores and germination medium immediately after combining may serve as a baseline fluorescence or a threshold value against which subsequent fluorescence intensity measurements can be compared. A fluorescence intensity which is greater than the baseline fluorescence during or after incubating the combination may indicate that viable spores are present. For certain embodiments, a fluorescence intensity which is at least 10 percent, preferably, at least 5 percent greater than the baseline indicates that viable spores are present.

For certain embodiments, including any one of the above method embodiments, whether or not as few as 100 viable spores are present is determined, and wherein the incubating is carried out for not more than 8 hours. For certain of these embodiments, preferably the incubating is carried out for not more than I hour. For certain of these embodiments, more preferably the incubating is carried out for not more than 30 minutes. For certain of these embodiments, even more preferably the incubating is carried out for not more than 15 minutes.

Alternatively, for certain embodiments, the method further comprises determining whether or not viable spores are present, after exposing the sterilization process indicator to a sterilant, by measuring a rate of the detectable change, if present, such as a rate at which fluorescence intensity changes. For example, after combining the spores with the germination medium and starting the incubating step, the detectable change may be measured continuously or intermittently over the incubation time and the rate of the detectable change determined. For certain embodiments, sterilization resistant spore germination may be detected as a rate of signal increase over the incubation time. This rate may be linear, exponential, or the like, with the respect to incubation time. The rate constant can be used as an indicator of germination of the spores.

Alternatively, for certain embodiments, the method further comprises determining whether or not viable spores are present, after exposing the sterilization process indicator to a sterilant, by taking a final fluorescence measurement at a specified time, such that if higher than a baseline fluorescence previously established for the product, the presence of viable spores is indicated. Higher than a baseline may be not more than 10 percent higher or not more than 5 percent higher. For example, after combining the spores with the germination medium and starting the incubating step, the final fluorescence may be measured at the end of incubation and used as an indicator of germination of the spores or of no germination.

The present method, which uses the compositions and indicators described above, can, therefore, be sufficiently sensitive to the presence of viable spores to provide an indication thereof in a short period of time. In addition, the indication can be provided even when the number of viable spores present is relatively low.

For certain embodiments, including any one of the above method embodiments, the method further comprises placing an article to be sterilized along with the sterilization process indicator in the sterilization chamber. For certain of these embodiments, the method further comprises determining whether or not the sterilization process was effective for sterilizing the article. An indication of no viable spores may be used to determine that the sterilization process was effective for sterilizing the article, whereas an indication of viable spores may be used to determine that the process was not effective. Thus, an assessment of the sterility of an article subjected to a sterilization process may be made in a relatively short time using the composition, indicator, and method embodiments described above by directly measuring production of nucleic acids in any viable spores.

Objects and advantages of this invention are further illustrated by the following examples, but the particular materials and amounts thereof recited in these examples, as well as other conditions and details, should not be construed to unduly limit this invention.

### EXAMPLES

### Spore Suspensions

Spore suspensions of *Gb. stearothermophilus* (also known as *Bacillus stearothermophilus*) were prepared by known methods as described in Example 1 of U.S. Patent No. 5,418,167 (Matner et al.). Modifications of these methods and alternative methods known to those skilled in the art may also be used for preparing these suspensions.

### Example 1

### Cell-Permeant Nucleic Acid-Interacting Fluorescent Dyes Detected Spore Germination

Representative cell-permeant dyes from the following classes: green fluorescent nucleic acid stains, red fluorescent nucleic acid stains, and Acridine Orange were tested with spores of *Gb stearothermophilus.* Specifically, 100 microliters of the spore suspension described above were combined with 200 microlitres of a 1: 5000 dilution of the dye with Sterile Water For Irrigation (Baxter, Deerfield, IL) or with nutrients glucose, fructose, and potassium chloride in water (1mg/ml, 1mg/ml, and 3.3 mgs/ml, respectively), 0.2 M valine , 0.04 M isoleucine, 0.2 M methionine, 4 mg/ml inosine, and 0.4 M alanine. The fluorescence intensity of each of the resulting suspensions was monitored at an incubation temperature of 50 °C, using the following dyes at the concentrations and excitation and emission wavelengths indicated below:
SYTO 24: 1.0 µM (micromolar); excitation at 485 nm, emission at 530 nm;
Acridine Orange: 6.6 µM; excitation at 485 nm, emission at 530 nm;
SYTO 64: 1.0 µM; excitation at 530 nm, emission at 620 nm.
The dyes are available from Invitrogen, (Carlsbad, CA). Fluorescence was monitored in a Biotek FL600 fluorimeter or Biotek Synergy 4 plate reader (Biotek, Winooski, VT). The results are shown in Table 1.

**Table 1. Fluorescence Intensity vs. Time of Spore Suspension with Dye and with Dye and Nutrients.**

| Time (min) | SYTO 24 | SYTO 24 With nutrients | SYTO 64 | SYTO 64 With nutrients | Acridine Orange | Acridine Orange With nutrients |
|---|---|---|---|---|---|---|
| 0 | 448 | 950 | 5060 | 3977 | 2005 | 2687 |
| 10 | 592 | 1376 | 4514 | 4913 | 2665 | 2777 |
| 20 | 697 | 2022 | 4254 | 6262 | 4069 | 4241 |
| 30 | 714 | 3653 | 4181 | 7660 | 5655 | 7842 |
| 40 | 754 | 4834 | 4218 | 8954 | 7091 | 11708 |

An increase in fluorescence intensity was found with each of the above dyes in the presence of the spores with nutrients.

### Example 2

The cell-permeant nucleic acid-interacting fluorescent dyes retained their ability to generate fluorescence in the presence of DNA and/or RNA within spores, after the dye was exposed to sterilization temperatures.

Dilutions of a *Gb. stearothermophilus* spore suspension described above were subjected to heat shock treatment at 80 °C for 10min. The dilutions were prepared using Sterile Water For Irrigation (Baxter, Deerfield, IL) and tested at a final population of 1x10⁶ and 0 spores. The spore dilutions (100 microlitres) were combined with 200 microlitres of medium, consisting of Acridine Orange and nutrients as described in Example 1, that had been subjected to a 15 min 121 °C (250 °F) vacuum assisted cycle in a AMSCO Scientific SG-120 Eagle/Century Series steam sterilizer (Steris, Mentor OH), and each of the resulting mixtures was added to a well of a plate. Un-autoclaved medium was used as a control. The plate was subsequently placed in a preheated plate reader (BioTek Synergy 4 plate reader, Winooski, VT) at 50 °C and incubated for 60 min, during which fluorescence intensity readings were taken using excitation at 485nm and emission at 530 nm. The experiment was performed with both heat shocked and non-heat shocked spores. A summary of the normalized data is listed below in Tables 2a-2c.

**Table 2a. Acridine Orange with Heat Shocked Spores of Gb.stearothermophilus.**

| Time (min) | Autoclaved Acridine orange | | Not Autoclaved Acridine orange | |
|---|---|---|---|---|
| | 0 spores | E6 germinating spores | 0 spores | E6 germinating spores |
| 0 | 0 | 0 | 0 | 0 |
| 30 | 522.25 | 6211.5 | 813 | 1623.75 |
| 60 | 588.75 | 8652.5 | 875.75 | 17121.25 |
| 90 | 779.75 | 9955.5 | 996.25 | 18928.25 |
| 120 | 870 | 10376.5 | 1099 | 19653.75 |
| 150 | 886.5 | 10694.25 | 1114.5 | 19884.5 |
| 180 | 960.5 | 10803 | 990.25 | 20008.25 |

**Table 2b. Acridine Orange with Non-heat Shocked Spores of Gb.sterothermophilus.**

| | Autoclaved Acridine orange | | Not Autoclaved Acridine orange | |
|---|---|---|---|---|
| Time (min) | 0 spores | E6 germinating spores | 0 spores | E6 germinating spores |
| 0 | 0 | 0 | 0 | 00 |
| 14 | -212 | 609 | -334 | 597 |
| 30 | -263 | 2599 | -456 | 2878 |
| 44 | -278 | 4753 | -484 | 5314 |
| 58 | -287 | 6508 | -476 | 7289 |

**Table 2c. SYTO 24 with Non-heat Shocked Spores of Gb. stearothermophilus.**

| Time (min) | Autoclaved SYTO 24 | | Not Autoclaved SYTO 24 | |
|---|---|---|---|---|
| | 0 spores | E6 spores | 0 spores | E6 spores |
| 0 | 0 | 0 | 0 | 0 |
| 14 | -33 | 1244 | -37 | 3039.5 |
| 30 | -29 | 3483 | -52 | 4704 |
| 44 | -53.5 | 4833 | -67 | 5060 |
| 58 | -41 | 5344 | -82 | 5259 |

The SYTO 24 was found to retain its ability to generate fluorescence even after being exposed to sterilization processes.

### Example 3

The cell-permeant nucleic acid-interacting fluorescent dyes had no more than a background level of fluorescence when subjected to a sterilization process which was just sufficient to decrease a population of at least 1xE6 spores to zero

A glass vial containing 1 mL of 1 x E7 *Gb.stearothermophilus* spore suspension described above and a glass vial containing 1 mL of medium containing a cell-permeant fluorescent dye as described in Example 1 were placed in a sterilizer and run in a 15 min 121 °C (250 "F) vacuum assisted cycle in a AMSCO Scientific SG-120 Eagle/Century Series steam sterilizer (Steris, Mentor OH), and 100 microlitres of the resulting autoclaved spore suspensions and medium were subsequently tested. Un-autoclaved spore suspensions that were subjected to a heat shock treatment at 80 °C for 10min were tested simultaneously as a control. Spore suspensions (100 microlitres) were combined with 200 microlitres of medium, consisting of Acridine orange dye and nutrients as described in Example 1, that was run through the same steam sterilization cycle as the spores, and each resulting mixture was added to a well of a plate. The plate was subsequently placed in a preheated plate reader (Synergy 4, BioTek, Winooski, VT) at 50 °C and incubated for 180 min, during which fluorescence intensity readings using excitation and emission wavelengths of 520 and 580, respectively were taken for at total of 19 readings. A summary of the normalized data is listed in Table 3.

**Table 3. Fluorescence Intensity vs Time of Spore Suspension with Live and Dead (Sterilized) Spores and with Acridine Orange Dye and Nutrient Medium Treated with the Same Sterilizing Conditions.**

| Time (min) | Autoclaved Spores | | Live Spores | |
|---|---|---|---|---|
| | 0 | E6 | 0 | E6 |
| 0 | 0 | 0 | 0 | 0 |
| 30 | 375.25 | -1468.25 | 522.25 | 6211.5 |
| 60 | 1347.25 | -103.5 | 588.75 | 8652.5 |
| 90 | 1533.75 | -9.25 | 779.75 | 9955.5 |
| 120 | 1491.25 | -49.25 | 870 | 10376.5 |
| 180 | 1427.75 | -307 | 886.5 | 10694.25 |

The results show that dead spores that were a result of the sterilization process did not have detectable levels of fluorescence in the presence of the cell-permeant fluorescent dye. Live spores on the other hand were capable of producing large amounts of measured fluorescence in the presence of the cell-permeant fluorescent dye.

### Example 4

The cell-permeant nucleic acid-interacting fluorescent dye based fluorescence was detected after exposure of *Gb.stearothermophilus* spores to a sub-lethal sterilization cycle.

Eighty biological indicators (BIs) were prepared by coating a suspension of *Gb.stearothermophilus spores* at a concentration of 1x E5 on a polypropylene carrier. Following drying at 37 °C for 20 min, the BIs were exposed to commonly used sterilization conditions as described below in Tables 4a, 4b, and 4c. Following sterilization the BIs were activated with a medium, exposed to the same sterilization conditions as the spores and containing of a 1: 5000 dilution of Acridine Orange along with glucose, fructose, and potassium chloride in water (1mg/ml, 1mg/ml, and 3.3 mgs/ml, respectively), 0.02 M alanine, 0.01 M valine, 0.002 M isoleucine, 0.2 mg/ml inosine, and a small amount of Bromo Cresol Purple. The combination of the medium with the spores on the carrier, subjected to lethal and sub-lethal sterilization processes shown in Tables 4a-4c, was incubated at 50 °C for 60 min, and fluorescence intensity readings were taken at 30 and 60 min using excitation at 485 nm and emission at 530 nm. Summaries of the changes in fluorescence following normalization are listed in Tables 4a-4c.

**Table 4a. Change in Acridine Orange Based Fluorescence (485/530 nm) at 30 and 60 Minute Intervals After 1 or 3 Minutes in a 132 °C (270 °F )Vaccuum Assisted Cycle in a Joslyn Steam Biological Indicator Evaluator Resistometer (BIER) Vessel (Steris, Mentor, OH).**

| Time in sterilizer | Average Change in Fluorescence at 30 min | Average Change in Fluorescence at 60 min |
|---|---|---|
| 1 min | 2607 | 2762 |
| 3 min | 279 | 386 |

**Table 4b. Change in Acridine Orange Based Fluorescence (485/530 nm) at 30 and 60 Minute Intervals After 1 or 3 Minutes in a 132 °C (270 °F) Gravity Cycle in a AMSCO EAGLE Model 2013 Steam Sterilizer (Steris, Mentor, OH).**

| Time in BIER vessel | Average Change in Fluorescence at 30 min | Average Change in Fluorescence at 60 min |
|---|---|---|
| 1 min | 1462 | 1808 |
| 3 min | 201 | 22 |

**Table 4c. Change in Acridine Orange Based Fluorescence (485/530 nm) at 30 and 60 Minute Intervals After 5 or 15 Minutes in a 121 °C (250 °F) Vacuum Assisted Cycle in a Joslyn Steam Biological Indicator Evaluator Resistometer (BIER) Vessel (Steris, Mentor, OH).**

| Time in BIER vessel | Average Change in Fluorescence at 30 min | Average Change in Fluorescence at 60 min |
|---|---|---|
| 5 min | 2774 | 3347 |
| 15 min | 383 | 353 |

All samples that were subjected to sub-lethal sterilization conditions (e.g., 1 min at 132 °C, 5 min at 121 °C) showed a significant increase in fluorescence intensity and turned the purple media to yellow, indicating growth and acid production, which provided independent evidence of viable spores.

### Example 5

The cell-permeant nucleic acid-interacting fluorescent dye showed activity over a range of dye concentrations in the solution.

Dilutions were prepared using Sterile Water For Irrigation (Baxter, Deerfield, IL) and tested at a final population ranging between of 1x10⁶ and 0 spores. The spore dilutions (100 microlitres) were combined with 200 microlitres of medium, consisting of Acridine Orange and nutrients as described in Example 1, with Acridine Orange concentrations at 1:5000 (0.0066mM), 1:1000 (0.033mM) and 1:500 (0.066mM), each of the resulting mixtures was added to a well of a plate. The plate was subsequently placed in a preheated plate reader (BioTek Synergy 4 plate reader, Winooski, VT) at 50 °C and incubated for 60 min, during which fluorescence intensity readings were taken using excitation at 485 nm and emission at 530 nm. A summary of the normalized data is listed below in Table 5a-5c.

**Table 5a. Acridine Orange at a concentration of 0.0066mM with decreasing concentrations of spores.**

| Time | 0 | E3 | E4 | E5 | E6 |
|---|---|---|---|---|---|
| 0:00:00 | 0 | 0 | 0 | 0 | 0 |
| 0:15:00 | 711 | 2033 | 3754.5 | 5224 | 12024.5 |
| 0:30:00 | 886.5 | 2271.5 | 4088 | 5364 | 15320 |
| 0:45:00 | 1027 | 2396 | 4174 | 5373 | 16514.5 |
| 1:00:00 | 1147.5 | 2484.5 | 4268 | 5406 | 17130.5 |

**Table 5b. Acridine Orange at a 0.033mM with decreasing concentrations of spores.**

| Time | 0 | E3 | E4 | E5 | E6 |
|---|---|---|---|---|---|
| 0:00:00 | 0 | 0 | 0 | 0 | 0 |
| 0:15:00 | 740 | 2022.5 | 3467 | 5012 | 14427.5 |
| 0:30:00 | 958 | 2281 | 3640.5 | 5029 | 19073.5 |
| 0:45:00 | 1143.5 | 2437 | 3724 | 4999 | 20802.5 |
| 1:00:00 | 1280 | 2558.5 | 3810.5 | 5068 | 21688 |

**Table 5c. Acridine Orange at 0.066mM with decreasing concentrations of spores.**

| Time | 0 | E3 | E4 | E5 | E6 |
|---|---|---|---|---|---|
| 0:00:00 | 0.0 | 0.0 | 0.0 | 0.0 | 0.0 |
| 0:15:00 | 815.5 | 3420.0 | 3261.5 | 3422.5 | 13727.5 |
| 0:30:00 | 1026.0 | 3716.0 | 3464.0 | 3533.0 | 15331.5 |
| 0:45:00 | 1184.5 | 3902.5 | 3586.5 | 3606.0 | 15529.5 |
| 1:00:00 | 1287.5 | 4000.5 | 3626.5 | 3616.0 | 15629.0 |

Detection of E3 spores was shown with all Acridine Orange concentrations. The higher the concentration of Acridine orange used, the higher the signal.

## Claims

1. A sterilization process indicator comprising:
a carrier supporting a plurality of sterilization process resistant spores;
a container impermeable to microorganisms and impermeable to a sterilant, the container containing a germination medium comprising a sub-lethal amount of at least one cell-permeant nucleic acid-interacting fluorescent dye and at least one nutrient for germination of the spores;
wherein the at least one cell-permeant fluorescent dye can interact with nucleic acids present in and produced by the plurality of spores during germination or during germination and outgrowth of the spores to produce an increase in fluorescence intensity, indicating that viable spores are present, and wherein the cell-permeant fluorescent dye is sufficiently stable at least at a temperature for incubating the spores to produce the increase in fluorescence intensity.
wherein the carrier is adjacent the container and separate from the germination medium.

2. The indicator of claim 1, wherein the cell-permeant fluorescent dye is sufficiently stable at a sterilization temperature.

3. The indicator of claim 1 or claim 2, wherein the cell-permeant fluorescent dye is a dye which interacts with the nucleic acids by intercalcation, electrostatic attraction, a charge interaction, hydrophobic-hydrophylic interactions, or a combination thereof.

4. The indicator of any one of claims 1 through 3, wherein the increase in fluorescence intensity is at a wavelength of 500 to 675 nm.

5. The indicator of any one of claims 1 through 4, wherein the germination medium further comprises a collisional quenching component.

6. The indicator of any one of claims 1 through 5, wherein the germination medium further comprises at least one reference dye.

7. A method of determining the effectiveness of a sterilization process, the method comprising:
providing a sterilization process indicator comprising:
a carrier supporting a plurality of sterilization process resistant spores;
a container impermeable to microorganisms and impermeable to a sterilant, the container containing a germination medium comprising a sub-lethal amount of at least one cell-permeant nucleic acid-interacting fluorescent dye and at least one nutrient for germination of the spores;
wherein the at least one cell-permeant fluorescent dye can interact with nucleic acids present in and produced by the plurality of spores during germination or during germination and outgrowth of the spores to produce an increase in fluorescence intensity, indicating that viable spores are present, and wherein the cell-permeant fluorescent dye is sufficiently stable at least at a temperature for incubating the spores to produce the increase in fluorescence intensity; and
wherein the carrier is adjacent the container and separate from the germination medium;
positioning the sterilization process indicator in a sterilization chamber;
exposing the sterilization process indicator to a sterilant;
combining the plurality of sterilization process resistant spores and the germination medium;
incubating the spores with the germination medium; and
measuring the increase in fluorescence intensity, if present.

8. The method of claim 7, further comprising determining whether or not viable spores are present, after exposing the sterilization process indicator to a sterilant, by measuring the increase in fluorescence intensity, if present, while incubating the spores with the germination medium and determining a rate of increase in fluorescence intensity, if present.

9. The method of claim 7, further comprising determining whether or not viable spores are present, after exposing the sterilization process indicator to a sterilant, by measuring the increase in fluorescence intensity, if present, after incubating the spores with the germination medium as compared with before incubating the spores with the germination medium.

10. The indicator of any one of claims 1-6 or the method of any one of claims 7 through 9, wherein the concentration of the cell-permeant fluorescent dye is not more than 0.10 mM.

## Patentansprüche

1. Sterilisationsvorgangsindikator, umfassend:
einen mehrere gegen Sterilisationsvorgänge resistente Sporen tragenden Träger;
einen für Mikroorganismen und ein Sterilisationsmittel undurchlässigen Behälter, der ein eine subletale Menge wenigstens eines zellgängigen, mit Nukleinsäure wechselwirkenden Fluoreszenzfarbstoff und wenigstens einen Nährstoff zur Keimung der Sporen umfassendes Keimungsmedium enthält,
wobei der wenigstens eine zellgängige Fluoreszenzfarbstoff mit in den mehreren Sporen während Keimung bzw. Keimung und Austreiben der Sporen vorliegenden und von diesen produzierten Nukleinsäuren wechselwirken kann, so dass ein Anstieg der Fluoreszenzintensität produziert wird, was das Vorliegen lebensfähiger Sporen anzeigt, und
wobei der zellgängige Fluoreszenzfarbstoff wenigstens bei einer Temperatur zur Inkubation der Sporen hinreichend stabil ist, um den Anstieg der Fluoreszenzintensität zu produzieren,
wobei der Träger an den Behälter angrenzend und vom Keimungsmedium getrennt vorliegt.

2. Indikator nach Anspruch 1, wobei der zellgängige Fluoreszenzfarbstoff bei einer Sterilisationstemperatur hinreichend stabil ist.

3. Indikator nach Anspruch 1 oder Anspruch 2, wobei es sich bei dem zellgängigen Fluoreszenzfarbstoff um einen Farbstoff handelt, der mit den Nukleinsäuren über Interkalation, elektrostatische Anziehung, eine Ladungswechselwirkung, hydrophobe-hydrophile Wechselwirkungen oder eine Kombination davon wechselwirkt.

4. Indikator nach einem der Ansprüche 1 bis 3, wobei der Anstieg der Fluoreszenzintensität bei einer Wellenlänge von 500 bis 675 nm vorliegt.

5. Indikator nach einem der Ansprüche 1 bis 4, wobei das Keimungsmedium ferner eine Kollisionsquencher-Komponente umfasst.

6. Indikator nach einem der Ansprüche 1 bis 5, wobei das Keimungsmedium ferner wenigstens einen Referenzfarbstoff umfasst.

7. Verfahren zur Bestimmung der Wirksamkeit eines Sterilisationsvorgangs, wobei man bei dem Verfahren:
einen Sterilisationsvorgangsindikator bereitstellt, umfassend:
einen mehrere gegen Sterilisationsvorgänge resistente Sporen tragenden Träger;
einen für Mikroorganismen und ein Sterilisationsmittel undurchlässigen Behälter, der ein eine subletale Menge wenigstens eines zellgängigen, mit Nukleinsäure wechselwirkenden Fluoreszenzfarbstoff und wenigstens einen Nährstoff zur Keimung der Sporen umfassendes Keimungsmedium enthält,
wobei der wenigstens eine zellgängige Fluoreszenzfarbstoff mit in den mehreren Sporen während Keimung bzw. Keimung und Austreiben der Sporen vorliegenden und von diesen produzierten Nukleinsäuren wechselwirken kann, so dass ein Anstieg der Fluoreszenzintensität produziert wird, was das Vorliegen lebensfähiger Sporen anzeigt, und
wobei der zellgängige Fluoreszenzfarbstoff wenigstens bei einer Temperatur zur Inkubation der Sporen hinreichend stabil ist, um den Anstieg der Fluoreszenzintensität zu produzieren, und
wobei der Träger an den Behälter angrenzend und vom Keimungsmedium getrennt vorliegt,
den Sterilisationsvorgangsindikator in eine Sterilisationskammer positioniert,
den Sterilisationsvorgangsindikator einem Sterilisationsmittel aussetzt,
die mehreren gegen Sterilisationsvorgänge resistenten Sporen und das Keimungsmedium zusammengibt,
die Sporen mit dem Keimungsmedium inkubiert
und
den Anstieg der Fluoreszenzintensität, falls vorhanden, misst.

8. Verfahren nach Anspruch 7, bei dem man ferner bestimmt, ob lebensfähige Sporen vorhanden sind oder nicht, nachdem der Sterilisationsvorgangsindikator einem Sterilisationsmittel ausgesetzt wurde, indem der Anstieg der Fluoreszenzintensität, falls vorhanden, während des Inkubierens der Sporen mit dem Keimungsmedium gemessen und eine Geschwindigkeit des Anstiegs der Fluoreszenzintensität, falls vorhanden, bestimmt wird.

9. Verfahren nach Anspruch 7, bei dem man ferner bestimmt, ob lebensfähige Sporen vorhanden sind oder nicht, nachdem der Sterilisationsvorgangsindikator einem Sterilisationsmittel ausgesetzt wurde, indem der Anstieg der Fluoreszenzintensität, falls vorhanden, nach Inkubieren der Sporen mit dem Keimungsmedium verglichen mit vor Inkubieren der Sporen mit dem Keimungsmedium gemessen wird.

10. Indikator nach einem der Ansprüche 1-6 oder Verfahren nach einem der Ansprüche 7 bis 9, wobei die Konzentration des zellgängigen Fluoreszenzfarbstoffs nicht mehr als 0,10 mM beträgt.

## Revendications

1. Indicateur de procédé de stérilisation, comprenant :
un support, supportant une pluralité de spores résistantes au procédé de stérilisation ;
un récipient imperméable aux microorganismes et imperméable à un stérilisant, le récipient contenant un milieu de germination comprenant une quantité sub-létale d'au moins un colorant fluorescent interagissant avec les acides nucléiques cellule-perméants, et au moins un nutriment pour la germination des spores ;
dans lequel le ou les colorants fluorescents cellule-perméants peuvent interagir avec des acides nucléiques présents dans la pluralité de spores et produits par cette pluralité pendant la germination ou pendant la germination et la croissance des spores, pour produire une augmentation de l'intensité de fluorescence, indiquant que des spores viables sont présentes, et dans lequel le colorant fluorescent cellule-perméant est suffisamment stable, au moins à une température permettant l'incubation des spores, pour produire une augmentation de l'intensité de fluorescence,
dans lequel le support est contigu au récipient et séparé du milieu de germination.

2. Indicateur de la revendication 1, dans lequel le colorant fluorescent cellule-perméant est suffisamment stable à une température de stérilisation.

3. Indicateur de la revendication 1 ou 2, dans lequel le colorant fluorescent cellule-perméant est un colorant qui interagit avec les acides nucléiques par intercalation, attraction électrostatique, une interaction de charges, des interactions hydrophobe-hydrophile ou une combinaison de celles-ci.

4. Indicateur de l'une quelconque des revendications 1 à 3, dans lequel l'augmentation de l'intensité de fluorescence a lieu à une longueur d'onde de 500 à 675 nm.

5. Indicateur de l'une quelconque des revendications 1 à 4, dans lequel le milieu de germination comprend en outre un composant d'extinction collisionnelle.

6. Indicateur de l'une quelconque des revendications 1 à 5, dans lequel le milieu de germination comprend en outre au moins un colorant de référence.

7. Procédé de détermination de l'efficacité d'un procédé de stérilisation, le procédé comprenant :
la mise à disposition d'un indicateur de procédé de stérilisation, comprenant :
un support, supportant une pluralité de spores résistantes au procédé de stérilisation ;
un récipient imperméable aux microorganismes et imperméable à un stérilisant, le récipient contenant un milieu de germination comprenant une quantité sub-létale d'au moins un colorant fluorescent interagissant avec les acides nucléiques cellule-perméants, et au moins un nutriment pour la germination des spores ;
dans lequel le ou les colorants fluorescents cellule-perméants peuvent interagir avec des acides nucléiques présents dans la pluralité de spores et produits par cette pluralité pendant la germination ou pendant la germination et la croissance des spores, pour produire une augmentation de l'intensité de fluorescence, indiquant que des spores viables sont présentes, et dans lequel le colorant fluorescent cellule-perméant est suffisamment stable, au moins à une température permettant l'incubation des spores, pour produire une augmentation de l'intensité de fluorescence ; et
dans lequel le support est contigu au récipient et séparé du milieu de germination ;
le positionnement de l'indicateur de procédé de stérilisation dans une chambre de stérilisation ;
l'exposition de l'indicateur de procédé de stérilisation à un stérilisant ;
la combinaison de la pluralité de spores résistantes au procédé de stérilisation et du milieu de germination ;
l'incubation des spores avec le milieu de germination ; et
la mesure de l'éventuelle augmentation de l'intensité de fluorescence.

8. Procédé de la revendication 7, comprenant la détermination si des spores viables sont présentes, ou non, après exposition de l'indicateur de procédé de stérilisation à un stérilisant, par mesure de l'éventuelle augmentation de l'intensité de fluorescence, tout en incubant les spores avec le milieu de germination et en déterminant un taux d'une éventuelle augmentation de l'intensité de fluorescence.

9. Procédé de la revendication 7, comprenant en outre la détermination si des spores viables sont présentes, ou non, après exposition de l'indicateur de procédé de stérilisation à un stérilisant, par mesure de l'éventuelle augmentation de l'intensité de fluorescence, après incubation des spores avec le milieu de germination, par comparaison avec avant l'incubation des spores avec le milieu de germination.

10. Indicateur de l'une quelconque des revendications 1 à 6 ou procédé de l'une quelconque des revendications 7 à 9, la concentration du colorant fluorescent cellule-perméant n'étant pas supérieure à 0,10 mM.
